(19) Europäisches Patentamt
European Patent Office
Office européen des brevets

(11) Veröffentlichungsnummer : **0 053 817**
**B1**

# (12) EUROPÄISCHE PATENTSCHRIFT

(45) Veröffentlichungstag der Patentschrift : **21.03.84**

(51) Int. Cl.³ : **C 07 C 85/06**, C 07 C 85/24

(21) Anmeldenummer : **81110158.3**

(22) Anmeldetag : **04.12.81**

## (54) Verfahren zur Herstellung von cycloaliphatischen Aminen.

(30) Priorität : **04.12.80 DE 3045719**

(43) Veröffentlichungstag der Anmeldung : **16.06.82 Patentblatt 82/24**

(45) Bekanntmachung des Hinweises auf die Patenterteilung : **21.03.84 Patentblatt 84/12**

(84) Benannte Vertragsstaaten : **AT CH DE FR GB IT LI NL SE**

(56) Entgegenhaltungen :
**DE-A- 1 468 779**
**DE-A- 2 045 882**
**FR-A- 1 427 543**
**FR-A- 1 506 010**

(73) Patentinhaber : **BASF Aktiengesellschaft**
**Carl-Bosch-Strasse 38**
**D-6700 Ludwigshafen (DE)**

(72) Erfinder : **Goetz, Norbert, Dr.**
**Schoefferstrasse 25**
**D-6520 Worms (DE)**
Erfinder : **Jacobs, Peter, Dr.**
**Kalmitstrasse 2**
**D-6718 Gruenstadt (DE)**
Erfinder : **Hupfer, Leopold, Dr.**
**Waltershoehe 3**
**D-6701 Friedelsheim (DE)**
Erfinder : **Toussaint, Herbert, Dr.**
**Knietschstrasse 11**
**D-6710 Frankenthal (DE)**
Erfinder : **Reiss, Wolfgang, Dr.**
**Bannwasserstrasse 70**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 053 817 B1

Jouve, 18, rue St-Denis, 75001 Paris, France

# Verfahren zur Herstellung von cycloaliphatischen Aminen

Die erfindung betrifft ein Verfahren zur Herstellung von cycloaliphatischen Aminen aus entsprechenden aromatischen Hydroxyverbindungen (Phenolen).

Die älteste und zur Zeit immer noch wichtigste Methode zur Herstellung cycloaliphatischer Amine ist die Reduktion der entsprechenden Nitroverbindungen (Houben-Weyl « Methoden der Org. Chemie », Band 11/1, S. 360 ff) zu primären aromatischen Aminen und deren Hydrierung. Nachteilig ist dabei die Tatsache, daß die Nitrierung bei substituierten Aromaten oft nicht einheitlich verläuft und fast stets Isomerengemische anfallen.

Prinzipiell gibt es auch die Möglichkeit, Phenole durch Partialhydrierung in Cyclohexanone zu überführen (DE-AS 1 124 487, DE-AS 1 298 098, DE-AS 1 144 267, US-PS 3 124 614, CH-PS 463 493, DE-OS 2 045 882) und die Cyclohexanone mit Ammoniak und Wasserstoff zu Cyclohexylaminen umzusetzen (Houben-Weyl, loc. cit., S. 611-617).

Diese Wege sind aber, da sie mehrere unabhängige Verfahrensschritte erfordern, recht umständlich und nicht besonders wirtschaftlich.

Die einstufige (direkte) Überführung von unsubstituiertem Phenol mit Ammoniak und Wasserstoff in Cyclohexylamin gelingt nach bisheriger Kenntnis in Gegenwart von Ruthenium- oder Rhodiumkatalysatoren (JA-OS 4 034 677, FR-PS 1 427 543, GB-PS 1 031 169, DE-PS12 76 032).

Nach einer nicht vorveröffentlichten Beschreibung ist 2,6-Dialkylphenol schon mit Ammoniak und Wasserstoff im Bereich eines bestimmten geringen Druckes (7 bis 21 bar) in das entsprechende Cyclohexylamin überführt worden, wobei Platin- oder Palladiumkatalysatoren empfohlen wurden (EP-A 22 751).

Schwierigkeiten bieten bisher offenbar besonders Verfahren, um mehrkernige Phenole vom Typus des Bisphenol A in entsprechende cycloaliphatische Polyamine zu überführen, die technisch wichtige Grundstoffe zur Herstellung von Polyurethanen und Polyamiden darstellen.

Solche Amine sind offenbar bisher nur über mindestens zweistufige Synthesewege erhalten worden (vgl. JA-Pat. Anm. 40 3960 und 70 21 702, DE-AS 14 68 779, DE-OS 20 55 620, FR 14 20 744).

Die Aufgabe, vor allem mehrkernige Amine aus entsprechenden Phenolen herzustellen, wird erfindungsgemäß im wesentlichen dadurch gelöst, daß man das Phenol mit Ammoniak und Wasserstoff an einem Edelmetall-(Platinmetall-) trägerkatalysator aus der Gruppe von Rhodium, Ruthenium, Palladium und Platin unter besonders hohem Druck — 50, vorzugsweise 100 bar oder mehr — umsetzt, wobei der Wasserstoffpartialdruck mindestens 20 bzw. vorzugsweise mindestens 50 bar betragen soll. Die Umsetzung findet somit im wesentlichen in der Flüssigphase statt.

Besonders wichtig ist ein hoher Wasserstoffdruck bei Verwendung von Palladiumkatalysatoren, die ausgeprägt dehydrierend auf cycloaliphatische Amine wirken.

Zur Gewinnung ein- oder mehrkerniger, cycloaliphatischer Amine zum Beispiel der allgemeinen Formel I

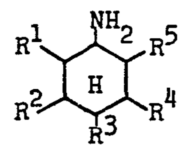

I,

in der $R^1$ bis $R^5$ jeweils ein Wasserstoffatom oder gleiche oder verschiedene oder jeweils zu mehreren gemeinsam einen Substituenten und insbesondere einen spiegelbildlichen Rest

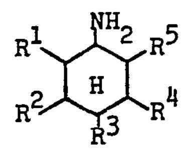

(Ia)

bedeuten können, wird demnach ein entsprechendes Phenol mit Ammoniak und Wasserstoff bei einer Temperatur zwischen etwa 100 und 300, vorzugsweise 100 bis 250 °C an einem Träger-Katalysator umgesetzt, der Palladium, Ruthenium, Rhodium oder Platin und vorzugsweise einen basischen Stoff und/oder ein Element aus der Gruppe 1 B, 2 B, 7 A des Periodensystems oder Eisen, Kobalt oder Nickel enthält.

Aliphatische Gruppen mit 1 bis 20 Kohlenstoffatomen, cycloaliphatische Gruppen mit 5 bis 20 Kohlenstoffatomen, Arylgruppen mit 6 bis 20 Kohlenstoffatomen oder Aralkyl- oder Alkylarylgruppen mit 7 bis 20 Kohlenstoffatomen sind Beispiele für derartige Substituenten. Diese können gegebenenfalls Sauerstoff und/oder Stickstoff als Heteroatome enthalten oder es können zwei Substituenten durch eine Molekülbrücke miteinander verbunden sein. Wenn ein Substituent dem ohne ihn verbleibenden Molekülrest entspricht, bedeutet die Formel I ein mehrkerniges, insbesondere zweikerniges Polyamin (Diamin).

Mit den erfindungsgemäßen Katalysatoren kann man an sich je nach Reaktionstemperatur und Wasserstoffdruck entweder cycloaliphatische oder aromatische Amine erhalten. Die Bildung von Nebenprodukten kann fast vollständig unterdrückt werden ; empfindliche Substituenten, wie Alkoxygruppen, werden im allgemeinen nicht angegriffen.

Dieser Befund ist überraschend, da nach dem Stande der Technik bei höherer Temperatur zahlreiche Nebenreaktionen und somit Nebenprodukte, wie Cyclohexanol, Cyclohexanon, Dicyclohexylamin, Phenyl-cyclohexylamin und Diphenylamin zu erwarten sind. Besonders gilt dies für die Gewinnung mehrkerniger, mehrfunktioneller Amine, im einfachsten Fall des 2,2-Bis-(4-aminocyclohexyl) propans aus Bisphenol A. Hier war das besondere Problem das Auftreten unvollständig umgesetzter Produkte, z. B. mit einem cycloaliphatischen und einem aromatischen Molekülteil, einer Amino- und einer Hydroxygruppe usw.

Die erhaltenen Cyclohexylamine lassen sich bei Bedarf leicht in Aniline überführen. Cyclohexylamine und entsprechende Aniline lassen sich im übrigen leicht trennen, so daß eine gewisse Menge an Anilinen ohne weiteres in Kauf genommen werden kann. Auf dieser Tatsache beruht ein wesentlicher Vorteil des hier beschriebenen Verfahrens, denn die als Ausgangsstoffe verwendeten Phenole lassen sich vollständig umsetzen.

Die als Ausgangsstoffe verwendeten Phenole sind im allgemeinen gut zugängliche Verbindungen (Houben-Weyl), Methoden, Band 6/1c),

Entsprechend der o. a. Formel für die Zielprodukte kann ein entsprechendes Phenol Substituenten $R^1$ bis $R^5$ haben, von denen einer oder mehrere auch selbst ein Phenol darstellen können, so daß also unter dem Begriff Phenol ein- oder mehrkernige Phenole zu verstehen sind. Bisphenol A ist ein Beispiel für ein mehrkerniges Phenol.

$R^1$ bis $R^5$ können ferner gleiche oder verschiedene Substituenten und selbstverständlich Wasserstoff bedeuten, wobei Alkyl- oder Aralkylreste mit jeweils 1 bis 20 C-Atomen Kettenlänge besonders wichtige Substituenten darstellen. Die Substituenten können außerdem Sauerstoff- oder Stickstoffatome in der Kette oder in heterocyclischer Anordnung aufweisen. Benachbarte Substituenten können zusammen mit dem Phenolrest einen Ring bilden, so daß als Phenole auch Naphthole und teilweise hydrierte Naphthole zu gelten haben.

Zwar ist ist die Umsetzung von Hydroxybenzol (« Phenol ») selbst nicht wirtschaftlich interessant, aber sie ist technisch möglich. Andere, substituierte Phenole sind etwa o-, m- und p-Kresol, o-Ethylphenol, o-n-Butylphenol, o-sek.-Butylphenol, 2,4-Dimethylphenol, 2,6-Dimethylphenol, 2,3,6-Trimethylphenol, 2,4,6-Trimethylphenol, 2-Cyclohexylphenol, 2,6-Dimethyl-3-phenyl-phenol, 2,6-Diethylphenol, 2,5-Diisopropylphenol, 2,6-Di-tert.-butylphenol, 2-Methyl-6-sek.-butylphenol, 3-tert.-Butylphenol, α-Naphthol, β-Naphthol, Bisphenol A (= 2,2-Di-(p-Hydroxyphenyl)-propan).

Der erforderliche Ammoniak kann — bezogen auf das eingesetzte Phenol — in stöchiometrischer Menge oder in einem, vorzugsweise 2 bis 10-fachen oder auch höheren Überschuß angewendet werden.

Das Verfahren kann kontinuierlich (fortlaufend) oder diskontinuierlich (absatzweise) durchgeführt werden. In jedem Falle kann der Katalysator in einem Festbett oder als Suspension angeordnet sein. Bevorzugt bei kontinuierlicher Arbeitsweise ist ein fest angeordneter Katalysator. Bei kontinuierlicher Arbeitsweise wird der Ammoniak zusammen mit dem Wasserstoff über den Katalysator geleitet. Im Falle diskontinuierlichen Betriebs führt man so lange Wasserstoff zu, bis das eingesetzte Phenol vollständig umgesetzt ist. Die Umsetzung kann ohne Lösungsmittel oder in Anwesenheit von Lösungsmitteln, die unter den Reaktionsbedingungen chemisch indifferent sind, durchgeführt werden.

Als Lösungsmittel kommen beispielsweise in Frage : Methanol, Ethanol, n-Butanol, Tetrahydrofuran, Dioxan, Cyclohexylmethylether, Methylglykol, Ethylglykol, 1,2-Dimethoxyethan, N,N-Dimethylcyclohexylamin, N-Methylpiperidin, N-Ethylpiperidin, N-Methylmorpholin, N-Methylpyrrolidin, Cyclohexan. In vielen Fällen ist das Zielprodukt selbst ein günstiges Lösungsmittel ; man führt einen Teilstrom zurück, wenn eine entsprechende Verdünnung gewünscht wird.

Das nach dem Verfahren der Erfindung verwendete Katalysatorsystem enthält Ruthenium, Rhodium, Palladium oder Platin auf einem Träger, wobei aus wirtschaftlichen Gründen Rhodium und Platin gegenwärtig weniger in Betracht kommen als die anderen Metalle.

Die Metalle können auf dem Träger allein verwendet werden, günstig ist aber die Verwendung gewisser aktiver Zusätze, nämlich Elementen bzw. Verbindungen von Elementen der Gruppen 1B, 2B und 7A des Periodensystems sowie Eisen, Kobalt und Nickel, unabhängig hiervon die Verwendung basischer Stoffe, d. h. Verbindungen der ersten bis dritten Hauptgruppe des Periodensystems. Die letzteren, basischen Stoffe bilden vorzugsweise einen Bestandteil des Trägers. Natürlich können die Zusätze allein oder im Gemisch miteinander neben dem Edelmetall vorliegen. Die aktiven Bestandteile des Katalysators sind auf einem Träger aufgebracht, der aus Aluminiumoxid oder einem unter Mitwirkung von Aluminiumoxid aufgebauten Stoff besteht. Aluminiumsilikat oder Spinelle des Aluminiums sind solche Stoffe. Die Zusätze, die dem Katalysator seine Eigenschaften verleihen, können sich an der Oberfläche des Katalysators befinden, zusammen mit dem übrigen Aluminiumoxid usw. in Form einer Mischung den Träger darstellen oder mit dem ursprünglichen Träger durch nachträgliches Tempern ein gemeinsames Kristallgitter bilden (beispielsweise bei Aluminiumoxidträgern Bildung von typischen Spinellgittern mit bestimmten Metallen). Das bedeutet, daß der Träger Aktivität und auch Lebensdauer des Katalysators günstig beeinflussen kann.

Als Zusätze kommen in Frage :

a) Basische Zusätze, wie Oxide, Hydroxide oder Carbonate der Alkalimetalle, vorzugsweise des Lithiums und Natriums, der Erdalkalimetalle, vorzugsweise des Magnesiums und Calciums und der Seltenerdmetalle, vorzugsweise des Cers und Praseodyms (Neodyms).

b) Weitere Metalle, wie Eisen, Nickel, Kobalt, Mangan, Zink, Cadmium und Silber.

Die Zusätze können gemeinsam mit dem Edelmetall durch Tränken des Trägermaterials mit Lösungen von z. B. Nitraten, Chloriden, Formiaten oder Oxalaten aufgebracht werden. Durch anschließende thermische Behandlung, die gewöhnlich zwischen 400 und 600 °C durchgeführt wird, erfolgt Überführung in die Oxidform. Soll bei Aluminiumoxidträgern mit geeigneten Metallen (Mg, Zn, Co, Mn, Li) Spinellbildung erzielt werden, muß nach der Tränkung auf eine Temperatur von 900-1 300 °C erhitzt werden (siehe Ullmanns Encyklopädie der technischen Chemie, 3. Auflage (1955) Band 6, S. 242-244, Gmelin, System-Nr. 35, Al, Tl, 1934-1935, S. 26-28) und anschließend in der üblichen Weise das Edelmetall aufgebracht werden.

Manche Zusätze, wie beispielsweise Calciumoxid oder Magnesiumoxid lassen sich mit einem Träger wie Aluminiumoxid mischen und bilden dann nach gemeinsamem Tempern bei 400-600 °C einen neuen Träger, auf dem das Edelmetall niedergeschlagen werden kann.

Der Edelmetallgehalt des Katalysators, bezogen auf das Trägermaterial, liegt gewöhnlich zwischen 0,05 bis 15 Gew.%.

Das Gewichtsverhältnis der Zusätze zum Edelmetall kann unterschiedlich sein ; es kann z. B. zwischen 10 000 : 1 bis 1 : 50, bevorzugt bei 100 : 1 bis 1 : 50 liegen. Man verwendet den Katalysator beispielsweise in Form von Strängen mit einem Durchmesser von 3 mm und einer Länge von 10 mm oder als Pulver, je nach dem vorgesehenen Anwendungszweck.

Cyclohexylamin und substituierte Cyclohexylamine finden Verwendung auf dem Textilhilfsmittelsektor, Pflanzenschutzmittelsektor, als Korrosionsschutzmittel und als Monomere für Kunststoffe (Ullmanns Encyklopädie der techn. Chemie, 3. Auflage, Band 5, Seiten 681-685).

Herstellmöglichkeiten für den Katalysator :

a) Auf strang- oder pulverförmiges µ-Aluminiumoxid wird das Edelmetall zusammen mit dem Zusatz (Mangan, Zink, Silber, Seltenerdmetalle u. a.) in der gewünschten Menge durch Tränken mit beispielsweise deren Nitratlösungen und anschließendes Eindampfen bis zur Trockne aufgebracht. Danach wird 6 Stunden bei 550 °C getempert und im Wasserstoffstrom bei 300 °C reduziert.

b) Auf strang- oder pulverförmiges µ-Aluminiumoxid wird zunächst die gewünschte Menge des Zusatzes (Mangan, Zink, Cobalt, Magnesium, Lithium u. a.) durch Tränken mit entsprechenden wäßrigen Nitrat- oder Formiatlösungen aufgebracht und bei 150 °C getrocknet. Der derart vorbehandelte Träger wird nun entweder 6 Stunden lang auf 550 °C erhitzt oder, wenn Spinellbildung erreicht werden soll, 6 Stunden lang bei 1050 °C geglüht. Nun wird der Träger mit wäßriger Edelmetallnitratlösung getränkt und durch 7-stündiges Erhitzen bei 300 °C im Wasserstoffstrom reduziert. Wurde zum Tränken eine Lösung eines Chlorids genommen, wird mit alkalischer Formalinlösung reduziert.

c) µ-Aluminiumoxid und ein basisches Oxid (MgO, CaO) werden in dem gewünschten Mengenverhältnis intensiv miteinander vermischt. Diese Mischung wird 6 Stunden lang auf 450 °C erhitzt, anschließend mit Edelmetallnitratlösung getränkt und 7 Stunden mit Wasserstoff bei 300 °C reduziert. Der Katalysator wird mit einer 5 %igen wäßrigen Hydrazinhydratlösung nachreduziert und dann bei 120 °C getrocknet.

Die in den nachfolgenden Beispielen angegebenen Siedepunkte beziehen sich, sofern nicht anders angegeben, auf atmosphärischen Druck, sonst auf verminderten Druck in mbar.

Beispiel 1

In ein zylindrisches Reaktionsrohr mit einem Volumen von 1 l wird ein Katalysator gefüllt, der aus 10 Gew.% Palladium, 5 Gew.% Praseodymoxid und im übrigen Aluminiumoxid in Form von Strängen (4 mm Durchmesser, 10 mm Länge) besteht. Bei 180 °C wird über diesen Katalysator stündlich eine Mischung aus 30 g 2,3,6-Trimethylphenol, 100 g 2,3,6-Trimethylcyclohexylamin und 1 000 g flüssigem Ammoniak geführt. Gleichzeitig werden 200 Nl Wasserstoff bei einem Druck von 250 bar im Gleichstrom durch das Reaktionsrohr hindurchgeleitet. Das abfließende Produkt wird unter Druck abgekühlt und dann entspannt. Es fallen hierbei stündlich ca. 130 g Rohprodukt an, die aus 2,3,6-Trimethylcyclohexylamin bestehen und ohne Reinigung weiter umgesetzt werden können ; zur kontinuierlichen Verfahrensgestaltung wird jeweils eine entsprechende Menge des Amins wieder zurückgeführt und mit frischem Phenol gemischt.

Beispiel 2

a) In einem 300 ml fassenden Rührautoklaven wird eine Mischung aus 54,5 g 2,3,6-Trimethylphenol, 41 g flüssigem Ammoniak und 6 g des in Beispiel 1 verwendeten Katalysators bei 230 °C und 250 bar 30 Stunden lang bei konstantem Druck hydriert. Das Reaktionsprodukt besteht nach gaschromatographischer Analyse aus 96 Gew.% 2,3,6-Trimethylcyclohexylamin und 4 Gew.% 2,3,6-Trimethylcyclohexanol.

b) Man verfährt wie vorstehend beschrieben bei einer Reaktionstemperatur von 250 °C. Das Reaktionsprodukt besteht dann aus 51 Gew.% 2,3,6-Trimethylcyclohexylamin, 3 Gew.% 2,3,6-Trimethylcyclohexanol und 46 Gew.% 2,3,6-Trimethylanilin.

c) Man setzt bei einer Reaktionstemperatur von 275 °C um :

Das Reaktionsprodukt besteht dann aus 29 Gew.% 2,3,6-Trimethylcyclohexylamin, 3 Gew.% 2,3,6-Trimethylcyclohexanol und 68 Gew.% 2,3,6-Trimethylanilin.

Beispiel 3

In einen 10 l-Rührautoklaven werden 1,650 kg

2,6-Dimethylphenol und 150 g eines pulverförmigen Katalysators, der 5,0 Gew.% Palladium, 2,5 Gew.% Praseodymoxid, Rest Aluminiumoxid, enthält, eingefüllt. Der Autoklav wird verschlossen und 1,370 kg Ammoniak aufgepreßt. Nun wird auf 230 °C erhitzt und durch Zufuhr von Wasserstoff ein Druck von 200 bar eingestellt. Man hält so lange auf Reaktionstemperatur, bis konstanter Druck erreicht ist (ca. 8 Stunden). Anschließend läßt man abkühlen, filtriert und erhält 1,710 kg rohes 2,6-Dimethylcyclohexylamin (Gemisch aus 3 stereoisomeren 2,6-Dimethylcyclohexylaminen). Man destilliert und erhält 1,685 kg 2,6-Dimethylcyclohexylamin, Kp = 167-168 °C, entsprechend einer Ausbeute von 98 %.

Beispiel 4

Man verfährt entsprechend Beispiel 3, bei 200 °C und mit einem Katalysator, der 0,5 Gew.% Palladium auf einem Gemisch aus 80,6 Gew.% Aluminiumoxid und 19,4 Gew.% Calciumoxid enthält. Auch mit diesem Katalysator wird 2,6-Dimethylcyclohexylamin in einer Ausbeute von 98 % bei vollständigem Umsatz erhalten.

Beispiel 5

Man verfährt entsprechend Beispiel 3, bei 180 °C und mit einem Katalysator, der 1,0 Gew.% Palladium auf einem Kobalt-Aluminium-Spinell als Träger enthält. 2,6-Dimethylcyclohexylamin fällt in einer Ausbeute von 94 % d. Th. an.

Beispiel 6

In einem 3 l-Rollautoklaven wird eine Mischung aus 550 g 2,3,6-Trimethylphenol und 510 g Ammoniak und im Gegenstrom 50 g Katalysator, der 5,0 Gew.% Palladium, 1,0 Gew.% Mangan und 5,0 Gew.% Silber auf Aluminiumoxid in Pulverform enthält, bei 220 °C und 200 bar Druck hydriert. Man kühlt ab, filtriert und destilliert. Man erhält 546 g 2,3,6-Trimethylcyclohexylamin, $Kp_{23}$ = 78-81 °C, entsprechend einer Ausbeute von 96 %.

Beispiel 7

Man verfährt entsprechend Beispiel 6 und erhält ausgehend von o-Kresol mit einem Katalysator, der 10 Gew.% Palladium, 0,11 Gew.% Zink, 0,1 Gew.% Cadmium auf Aluminiumoxid enthält, 2-Methylcyclohexylamin, Kp = 147 °C, in einer Ausbeute von 98 %.

Beispiel 8

Entsprechend Beispiel 6 wird Phenol an einem Katalysator aus 5,0 Gew.% Palladium, 2,5 Gew.% Ce-IV-oxid auf Aluminiumoxid umgesetzt. Man erhält in einer Ausbeute von 95 % Cyclohexylamin, Kp = 134 °C.

Beispiel 9

In einem 300 ml-Rührautoklaven wird eine Mischung aus 46 g Bisphenol A (2,2-Bis-(4-hydroxyphenyl)-propan, 41 g Ammoniak und 6 g Katalysator (10 Gew.% Palladium, 5 Gew.% Praseodymoxid auf Aluminiumoxid) bei 200 °C und 250 bar hydriert. Man nimmt in Methanol auf, filtriert, destilliert das Lösungsmittel bei Normaldruck ab und destilliert den Rückstand unter vermindertem Druck. Hierbei fallen 45 g 2,2-Bis-(4-aminocyclohexyl)-propan, $Kp_{0,4}$ = 144-147 °C an, entsprechend einer Ausbeute von 94 %.

Beispiel 10

Man verfährt entsprechend Beispiel 9, setzt aber 2,6-Dimethyl-3-phenyl-phenol um, indem zum Reaktionsgemisch noch 5 g gesättigte wäßrige Sodalösung und ein Katalysator aus 0,5 Gew.% Palladium auf Aluminiumoxid zugegeben werden. In einer Ausbeute von 95 % wird 2,6-Dimethyl-3-cyclohexylcyclohexylamin ($Kp_{0,2}$ = 95-98 °C) erhalten.

Beispiel 11

Man verfährt entsprechend Beispiel 9, verwendet aber als Ausgangsprodukt 2,2-Dimethyl-5-hydroxychroman und einen Katalysator der Zusammensetzung 5,0 Gew.% Palladium, 1,0 Gew.% Mangan auf Aluminiumoxid. 2,2-Dimethyl-5-amino hexahydrochroman ($Kp_{27}$ = 128-131 °C) wird in einer Ausbeute von 97 % erhalten.

Beispiel 12

Man verfährt analog Beispiel 9, verwendet aber als Ausgangsprodukt 2-Ethylphenol und einen Katalysator der Zusammensetzung 1,0 Gew.% Palladium auf einem Lithium-Aluminium-Spinell. 2-Ethylcyclohexylamin ($Kp_{27}$ = 68-70 °C) wird in einer Ausbeute von 95 % erhalten.

Beispiel 13

Man verfährt analog Beispiel 9, verwendet aber als Ausgangsprodukt 3-tert.-Butylphenol und einen Katalysator mit der Zusammensetzung 1,0 Gew.% Palladium, 0,5 Gew.% Praseodymoxid auf Aluminiumoxid. 3-tert.-Butylcyclohexylamin ($Kp_{27}$ = 84-85 °C) wird in einer Ausbeute von 97 % erhalten.

Beispiel 14

In ein zylindrisches Reaktionsrohr mit einem Volumen von 1 l wird ein Katalysator aus 1,0 Gew.% Palladium, 0,5 Gew.% Praseodymoxid, Rest Aluminiumoxid, in Strangform eingefüllt und auf 200 °C erhitzt. Über dieses Katalysatorbett wird stündlich eine Mischung aus 60 g 2,6-Dimethylphenol und 360 g flüssigem Ammoniak geführt. Gleichzeitig werden 100 Nl Wasserstoff bei einem Druck von 200 bar durch das Reaktionsrohr im

Gleichstrom hindurchgeleitet. Das ablaufende Reaktionsprodukt wird unter Druck gekühlt und dann entspannt. Es fallen hierbei stündlich ca. 62 g Rohprodukt an, die nach Destillation 60 g reines 2,6-Dimethylcyclohexylamin (entsprechend einer Ausbeute von 96 %) ergeben.

Beispiel 15

In einem 300 ml-Rührautoklaven wird eine Mischung aus 35 g Bisphenol A (2,2-Bis-(4-hydroxyphenyl)-propan), die in 60 ml Tetrahydrofuran gelöst sind, 41 g Ammoniak und 5 g Katalysator (Zusammensetzung : 0,5 Gew.-% Ruthenium auf Magnesium-Aluminiumspinell) bei 210 °C und 250 bar Gesamtdruck bis zur Druckkonstanz (ca. 8 Stunden) hydriert. Das Reaktionsprodukt wird filtriert, das Lösungsmittel bei Normaldruck abdestilliert und man erhält als Rückstand 35,5 g 2,2-Bis-(4-aminocyclohexyl)-propan, $Kp_{0,4} = 144$ bis 147 °C, entsprechend einer Ausbeute von 97 % d. Th.

Beispiel 16

Man verfährt analog Beispiel 15, verwendet aber eine Katalysator, der 0,5 Gew.-% auf Lithium-Aluminiumspinell enthält. Ausgehend von m-(4-Methyl-tetrahydropyran-2-yl)-phenol erhält man 3-(4'-Methyl-tetrahydropyran-2'-yl)-cyclohexylamin ($Kp_{0,13} = 80$ bis 81 °C) in einer Ausbeute von 95 % d. Th.

Beispiel 17

Man verfährt analog Beispiel 15, verwendet aber einen Katalysator, der 0,5 Gew.-% Rhodium auf Magnesium-Aluminiumspinell enthält. Ausgehend von 2-Methyl-6-tert.-butyl-phenol erhält man 2-Methyl-6-tert.-butyl-cyclohexylamin (Kp = 220 bis 221 °C) in einer Ausbeute von 95 % d. Th.

Beispiel 18

Man verfährt analog Beispiel 15, verwendet aber einen Katalysator, der 0,5 Gew.-% Ruthenium auf einem Träger enthält, der durch mechanisches Vermischen von 19,4 Gew.-% Magnesiumoxid und 80,6 Gew.-% Aluminiumoxid erhalten wird. Ausgehend von 2-sec.-Butyl-phenol erhält man 2-sec.-Butylcyclohexylamin (Kp = 212 bis 213,5 °C) in einer Ausbeute von 96 % d. Th.

Beispiel 19

Man verfährt analog Beispiel 15 verwendet aber einen Katalysator, der 1,5 Gew.-% Ruthenium auf Zink-Aluminium-spinell enthält. Ausgehend von 2,6-Di-isopropylphenol erhält man als Reaktionsprodukt 2,6-Di-isopropyl-cyclohexylamin (Kp = 239 bis 240 °C) in einer Ausbeute von 93 % d. Th.

Beispiel 20

In ein druckfestes zylindrisches Rohr mit einem Volumen von 1 l als Reaktor wird ein Katalysator in Form von Strängen (3 mm Durchmesser, 10 mm Länge), der 0,5 Gew.-% Ruthenium auf Aluminiumoxid als Träger enthält, eingefüllt und auf 230 °C erhitzt. Bei dieser Temperatur wird über diesen Katalysator bei einem Gesamtdruck von 250 bar stündlich eine Mischung aus 10 g Bisphenol A, die in 100 g Triisobutylamin gelöst sind und 300 g flüssigem Ammoniak geführt. Gleichzeitig werden 200 Nl/h Wasserstoff durch das Reaktionsrohr hindurchgeleitet. Das abfließende Produkt wird unter Druck abgekühlt und dann entspannt. Es fallen hierbi stündlich ca. 110 g Rohprodukt an. Nach Entfernen des Lösungsmittels unter vermindertem Druck erhält man 10,2 g reines 2,2-Bis-(4-aminocyclohexyl)-propan, $Kp_{0,4} = 144$ bis 147 °C, entsprechend einer Ausbeute von 98 % d. Th. Zur kontinuierlichen Verfahrensgestaltung werden unverbrauchter Wasserstoff und Ammoniak im Kreis geführt.

**Ansprüche**

1. Verfahren zur Herstellung von cycloaliphatischen Aminen der allgemeinen Formel $RNH_2$, in der R einen gegebenenfalls substituierten oder annellierten Cycloalkylrest bedeutet, durch Umsetzung von entsprechenden aromatischen Hydroxyverbindungen (Phenolen) mit Ammoniak und Wasserstoff in Gegenwart eines Katalysators bei erhöhter Temperatur und erhöhtem Druck, dadurch gekennzeichnet, daß man die Umsetzung in Gegenwart eines auf einem Träger aufgebrachten Ruthenium-, Rhodium-, Palladium- oder Platinkatalysators durchführt und einen Wasserstoffpartialdruck von wenigstens 20 bar bzw. einen Gesamtdruck von wenigstens 50 bar einhält.

2. Verfahren nach Anspruch 1, dadurch gekennzeichnet, daß man einen Katalysator verwendet, der einen basischen Stoff und/oder ein Element aus der Gruppe 1 B, 2 B, 7 A des Periodensystems, Eisen, Kobalt oder Nickel enthält.

3. Verfahren gemäß Anspruch 2, dadurch gekennzeichnet, daß der Katalysator als basischen Stoff ein Oxid, Hydroxid oder Carbonat eines Alkalimetalls oder eines Erdalkalimetalls enthält.

4. Verfahren gemäß Anspruch 2, dadurch gekennzeichnet, daß der Katalysator als basischen Stoff ein Oxid, Hydroxid oder Carbonat eines Seltenerdmetalls enthält.

5. Verfahren nach Anspruch 3, dadurch gekennzeichnet, daß der Katalysator eine Magnesiumverbindung enthält.

6. Verfahren nach Anspruch 4, dadurch gekennzeichnet, daß der Katalysator eine Praseodym/Neodym- oder Cerverbindung enthält.

**Claims**

1. A process for the preparation of cycloaliphatic amines of the general formula $RNH_2$, where R

is an optionally substituted or fused cycloalkyl, by reacting appropriate aromatic hydroxy compounds (phenols) with ammonia and hydrogen in the presence of a catalyst at elevated temperature and pressure, wherein the reaction is carried out in the presence of a supported ruthenium, rhodium, palladium or platinum catalyst, and a hydrogen partial pressure of at least 20 bars or a total pressure of at least 50 bars is maintained.

2. A process as claimed in claim 1, wherein a catalyst is used which contains a basic substance and/or an element selected from groups 1B, 2B or 7B of the periodic table, iron, cobalt or nickel.

3. A process as claimed in claim 2, wherein the catalyst contains, as basic substance, an oxide, hydroxide or carbonate of an alkali metal or alkaline earth metal.

4. A process as claimed in claim 2, wherein the catalyst contains, as basic substance, an oxide, hydroxide or carbonate of a rare earth metal.

5. A process as claimed in claim 3, wherein the catalyst contains a magnesium compound.

6. A process as claimed in claim 4, wherein the catalyst contains a praseodymium/neodymium or cerium compound.

**Revendications**

1. Procédé pour la préparation d'amines cycloaliphatiques de formule générale $RNH_2$ (dans laquelle R représente un radical cycloalcoyle éventuellement substitué ou à noyaux condensés), par réaction de composés hydroxylés aromatiques (phénols) correspondant avec l'ammoniac et l'hydrogène en présence d'un catalyseur, à haute température et sous une pression élevée, caractérisé en ce qu'on mène la réaction en présence d'un catalyseur de ruthénium, de rhodium, de palladium ou de platine fixé sur un support, et en ce qu'on maintient une pression partielle d'hydrogène d'au moins 20 bars ou une pression totale d'au moins 50 bars.

2. Procédé selon la revendication 1, caractérisé en ce qu'on utilise un catalyseur qui contient une matière basique et/ou un élément des groupes 1B, 2B, 7A du système périodique, du fer, du cobalt ou du nickel.

3. Procédé selon la revendication2, caractérisé en ce que le catalyseur contient, en tant que matière basique, un oxyde, hydroxyde ou carbonate d'un métal alcalin ou d'un métal alcalinoterreux.

4. Procédé selon la revendication 2, caractérisé en ce que le catalyseur contient, en tant que matière basique, un oxyde, hydroxyde ou carbonate d'un métal des terres rares.

5. Procédé selon la revendication 3, caractérisé en ce que le catalyseur contient un composé du magnésium.

6. Procédé selon la revendication 4, caractérisé en ce que le catalyseur contient un composé du praséodyme/néodyme ou du cérium.